# EUROPÄISCHE PATENTSCHRIFT

(11) **EP 0 561 298 B1**
(45) Veröffentlichungstag und Bekanntmachung des Hinweises auf die Patenterteilung: **02.08.1995**
(21) Anmeldenummer: 93104064.6
(22) Anmeldetag: 12.03.1993
(51) Int. Cl.: C07C 201/12, C07C 205/26

(54) **Verfahren zur Herstellung von 2-Nitro-3,6-dichlorphenol**
Process for the preparation of 2-Nitro-3,6-Dichlorophenol
Procédé pour la préparation de nitro-2-dichloro-3,6-phénol

(30) Priorität: 18.03.1992 DE 4208630
(43) Veröffentlichungstag der Anmeldung: 22.09.1993
(73) Patentinhaber: HOECHST AKTIENGESELLSCHAFT, 65926 Frankfurt am Main (DE)
(72) Erfinder: Meier, Michael, Dr., W-6000 Frankfurt/M. 90 (DE); Kautz, Heinz-Georg, W-6484 Birstein (DE)

(56) Entgegenhaltungen:
- DE-B- 2 501 899
- US-A- 5 012 015
- JOURNAL OF THE CHEMICAL SOCIETY. Dezember 1929, GB Seiten 2917 - 2923 HERBERT HENRY HODGSON ET AL. 'Halogenation of Anisole Derivatives'
- JOURNAL OF HETEROCYCLIC CHEMISTRY Bd. 4, 1967, PROVO US Seiten 611 - 618 H.M. GROTTA ET AL. 'Some Hydroxylated Derivatives of Chlorpromazine'

## Beschreibung

Die vorliegende Erfindung betrifft ein verbessertes Verfahren zur Herstellung von 2-Nitro-3,6-dichlorphenol durch Sulfonierung von 2,5-Dichlorphenol mit anschließender Nitrierung und Desulfonierung.

2-Nitro-3,6-dichlorphenol ist ein wertvolles Zwischenprodukt für die Herstellung von Pharmazeutika.

In der DE-A-25 01 899 ist die Sulfonierung von 4-Chlor-5-methylphenol zu 4-Chlor-5-methylphenol-2-sulfonsäure, in der US-A-5,012,015 ist die Nitrierung von Trichloralkylbenzolen beschrieben.

In J. Chem. Soc. 1929, 2917-2923 (H.H. Hodgson, A. Kershaw) wurde beschrieben, daß man 2,5-Dichlorphenol mit Oleum sulfonieren, anschließend mit einem Gemisch aus Salpetersäure und Oleum nitrieren und dann die Sulfonsäuregruppe wieder abspalten kann. Die Angaben zu den Reaktionsparametern sind jedoch unvollständig, so daß dieses Verfahren nicht nachvollziehbar ist. Auch wird hier die Salpetersäure in weit unterstöchiometrischem Verhältnis eingesetzt.

Von H.M. Grotta, T.F. Page Jr., J. Riggle, A.A. Manian, J. Heterocycl. Chem. 4, 611-618 (1967) wurde bereits erkannt, daß das vorgenannte Verfahren in der dort geschilderten Form nicht durchführbar ist. Die Autoren veränderten dieses Verfahren dahingehend, daß sie 2-Nitro-3,6-dichlorphenol durch Sulfonierung mit Oleum bei 80-90°C und anschließender Nitrierung durch Zugabe eines Gemisches aus Salpetersäure mit Oleum in 30 min bei 0°C und Nachreaktion von 17 h bei 25°C und weitere 5 h bei 60°C herstellten. Nach anschließender Desulfonierung erhielten sie das Produkt in 51 %iger Ausbeute. Nach wie vor nachteilig an diesem Verfahren sind die Verwendung von Oleum auch bei der Nitrierung, die sehr langen Reaktionszeiten und die niedrigen Ausbeuten.

Weiter bekannt sind die photochemische Umsetzung von p-Dichlorbenzol mit NO (K. Nojima, K.Sauro, Chemosphere 9, 437-440 (1980)) und die Umsetzung von Phenol mit Nitrylchlorid in Methylenchlorid bei -50°C (M.J. Collis, D.R. Goddard, J. Chem. Soc. 1958, 1952-1955). Beide Umsetzungen sind technisch jedoch nicht verwertbar, da das 2-Nitro-3,6-dichlorphenol in geringer Ausbeute und im Gemisch mit anderen Isomeren entsteht.

Es bestand daher ein Bedürfnis nach einem wirtschaftlichen und technisch leicht durchführbaren Verfahren zur Herstellung von isomerenfreiem 2-Nitro-3,6-dichlorphenol in guten Ausbeuten, das vorzugsweise ohne die Verwendung von Oleum auskommt.

Es wurde nun gefunden, daß man isomerenfreies 2-Nitro-3,6-dichlorphenol in hohen Ausbeuten herstellen kann, indem man sulfoniertes 2,5-Dichlorphenol durch Zudosieren einer Nitriersäure aus Salpetersäure und Schwefelsäure über mindestens 1 h nitriert, wobei die Temperatur während der Nitrierung unter 35°C gehalten wird, und anschließend desulfoniert.

Gegenstand der Erfindung ist somit ein Verfahren zur Herstellung von 2-Nitro-3,6-dichlorphenol durch Sulfonierung von 2,5-Dichlorphenol und anschließende Nitrierung und Desulfonierung, das dadurch gekennzeichnet ist, daß man die Nitrierung durch Zudosierung einer Nitriersäure aus 98 bis 100 %iger, vorzugsweise 100 %iger Salpetersäure und 96 bis 100 %iger, vorzugsweise 100 %iger Schwefelsäure bei Temperaturen von -15 bis 35°C und einer Dosierzeit von mindestens 1 h durchführt.

Im allgemeinen wird die Schwefelsäure in der 1,5 bis 10fachen, bevorzugt der 2 bis 8fachen, besonders bevorzugt der 4 bis 6fachen, Gewichtsmenge, bezogen auf die Salpetersäure, eingesetzt. Die Salpetersäure selbst wird zweckmäßig in einer Menge von 0,8 bis 1,3 mol, vorzugsweise in einer Menge von 0,9 bis 1,2 mol und besonders bevorzugt in einer Menge von 1,0 bis 1,1 pro mol 2,5-Dichlorphenol eingesetzt.

Bei dem erfindungsgemäßen Verfahren wird die Nitrierung bei Temperaturen von -15 bis 35°C durchgeführt. Höhere Temperaturen führen zunehmend zu unerwünschten Dinitierungen. Auch durch eine zu schnelle Zudosierung der Nitriersäure nehmen Dinitrierungen zu. Vorzugsweise wird die Nitrierung bei Temperaturen von 0 bis 25°C, zweckmäßig bei 5 bis 20°C, durchgeführt. Die Zeit, in der die Nitriersäure zugesetzt wird beträgt vorteilhaft mindenstens 1,5 h, bevorzugt jedoch mindestens 2 h. Weit längere Zeiten als ca. 10 h sind in der Praxis jedoch nicht sinnvoll.

Die Sulfonierung und die Desulfonierung können in an sich bekannter Weise durchgeführt werden. Es ist beispielsweise möglich mit Oleum zu sulfonieren. Bevorzugt wird jedoch die Sulfonierung mit 98 bis 100 %iger, vorteilhaft mit 100 %iger Schwefelsäure bei Temperaturen von 70 bis 130°C, durchgeführt. Dabei wird die Schwefelsäure bzw. das Oleum zweckmäßigerweise in der etwa 2,5 bis 12fachen, vorzugsweise in der 4 bis 8fachen, Gewichtsmenge, bezogen auf 2,5-Dichlorphenol, eingesetzt.

Im allgemeinen wird so verfahren, daß man 2,5-Dichlorphenol mit 100 %iger Schwefelsäure versetzt und das Reaktionsgemisch bei 70 bis 130°C ca. 1 h nachrührt. Man kann aber auch das 2,5-Dichlorphenol zu der vorgelegten Schwefelsäure zudosieren. Nach anschließender Nitrierung wird desulfoniert, indem man beispielsweise das Reaktionsgemisch mit Wasser versetzt, gegebenenfalls bei Temperaturen von ca. 150 bis 170°C wasserdampfdestilliert und das überdestillierende 2-Nitro-3,6-dichlorphenol isoliert. Es ist aber auch möglich, nach beendeter Nitrierung die Reaktionsmischung mit Wasser zu versetzen, ca. 4 h auf ca. 150 bis 170°C zu erhitzen, unter Rühren abzukühlen und das ausgefallene Produkt ohne Wasserdampfdestillation zu isolieren.

Es ist überraschend, daß bei dem erfindungsgemäßen Verfahren, trotz Verwendung von weniger reaktiver Nitriersäure als im Stand der Technik, die Nitrierzeiten deutlich von über 20 h auf unter 5 h reduziert werden können, wobei die Ausbeuten und Reinheiten des gebildeten 2-Nitro-3,6-dichlorphenols drastisch verbessert werden. Auch der Vorteil, daß es das erfindungsgemäße Verfahren ermöglicht, die Sulfonierung mit Schwefelsäure anstelle von Oleum durchzuführen, war nicht zu erwarten.

### Beispiele

1. In einem 1l-Vierhalskolben mit Rührer, Innenthermometer und Tropftrichter wurden 81,5 g (0,5 mol) 2,5-Dichlorphenol vorgelegt, mit 222 g 100 %iger Schwefelsäure versetzt und 1 h bei 100°C nachgerührt. Das Reaktionsgemisch wurde auf 15°C abgekühlt und über 2 h eine Mischung aus 33,1 g (0,525 mol) 100 %iger Salpetersäure und 165,5 g 100 %iger Schwefelsäure bei 15°C zudosiert. Anschließend wurde 30 min bei 15°C nachgeführt, das Reaktionsgemisch mit 100 ml Wasser versetzt und bei 150 bis 170°C wasserdampfdestilliert. Es gehen insgesamt 980 g Destillat über. Das im Destillat ausgefallene 2-Nitro-3,6-dichlorphenol wurde abfiltriert. Es wurden 99,3 g feuchtes Produkt mit einem Wassergehalt von 8,8 % und einer Reinheit von 98,2 % (nach GC, Silyierung mit BSTFA) erhalten. Dies entspricht einer Ausbeute von 85,5 % (100 %ig gerechnet). Der Schmelzpunkt betrug 48°C.
   - ¹H-NMR (CDCl₃): δ =: 7,05 (d, ³J_{H,H} = 10 Hz; 1 H; ArH), 7,45 (d, ³J_{H,H} = 10 Hz; 1 H; ArH), 7,9 (breit; 1 H; OH).
2. 122,25 g (0,75 mol) 2,5-Dichlorphenol wurden mit 500 g 100 %iger Schwefelsäure versetzt und 1 h bei 100°C nachgerührt. Anschließend wurde auf 0°C abgekühlt und über 2 h eine Mischung aus 49,6 g (0,79 mol) 100 %iger Salpetersäure und 246,5 g 100 %iger Schwefelsäure bei 0°C zudosiert.
   Anschließend wurde das Reaktionsgemisch mit 100 ml Wasser versetzt und bei 150 bis 170°C wasserdampfdestilliert. Es gehen insgesamt 1854 g Destillat über. Das im Destillat ausgefallene 2-Nitro-3,6-dichlorphenol wurde abfiltriert. Es wurden 152,4 g feuchtes Produkt mit einem Wassergehalt von 10,5 % und einer Reinheit von 97,5 % (nach GC) erhalten. Dies entspricht einer Ausbeute von 86,1 % (100 %ig gerechnet). Der Schmelzpunkt betrug 46°C und die spektroskopischen Daten waren identisch mit denen aus Beispiel 1.
3. 122,25 g (0,75 mol) 2,5-Dichlorphenol wurden mit 555 g 100 %iger Schwefelsäure versetzt und 1 h bei 120°C nachgerührt. Anschließend wurde auf 0°C abgekühlt und über 2 h eine Mischung aus 49,6 g (0,79 mol) 100 %iger Salpetersäure und 246,5 g 100 %iger Schwefelsäure bei 0°C zudosiert.
   Anschließend wurde das Reaktionsgemisch mit 100 ml Wasser versetzt und bei 150 bis 170°C wasserdampfdestilliert. Es gehen insgesamt 1263 g Destillat über. Das im Destillat ausgefallene 2-Nitro-3,6-dichlorphenol wurde abfiltriert. Es wurden 151,1 g feuchtes Produkt mit einem Wassergehalt von 9,2 % und einer Reinheit von 96,0 % (nach GC) erhalten. Dies entspricht einer Ausbeute von 85,3 % (100 %ig gerechnet). Der Schmelzpunkt betrug 47°C und die spektroskopischen Daten waren identisch mit denen aus Beispiel 1.
4. 122,25 g (0,75 mol) 2,5-Dichlorphenol wurden mit 500 g 100 %iger Schwefelsäure versetzt und 1 h bei 100°C nachgerührt. Anschließend wurde auf 15°C abgekühlt und über 2 h eine Mischung aus 49,6 g (0,79 mol) 100 %iger Salpetersäure und 246,5 g 100 %iger Schwefelsäure bei 15°C zudosiert.
   Anschließend wurde das Reaktionsgemisch mit 250 ml Wasser versetzt und 4 h auf 150-160°C erhitzt. Nach dem Abkühlen wurde das Reaktionsgemisch auf Eis gegeben und das ausgefallene Produkt abgesaugt. 2-Nitro-3,6-dichlorphenol wurde nochmals in 250 ml Wasser aufgeschlämmt und erneut abgesaugt. Es wurden 150,0 g feuchtes Produkt mit einem Wassergehalt von 2,5 % und einer Reinheit von 93,8 % (nach GC) erhalten. Dies entspricht einer Ausbeute von 87,9 % (100 %ig gerechnet). Der Schmelzpunkt betrug 42°C und die spektroskopischen Daten waren identisch mit denen aus Beispiel 1.
5. 122,25 g (0,75 mol) 2,5-Dichlorphenol wurden mit 640 g 100 %iger Schwefelsäure versetzt und 1 h bei 100°C nachgeführt. Anschließend wurde auf 15°C abgekühlt und über 4 h eine Mischung aus 48,3 g (0,766 mol) 100 %iger Salpetersäure und 96,6 g 100 %iger Schwefelsäure bei 10-15°C zudosiert.
   Anschließend wurde das Reaktionsgemisch mit 100 ml Wasser versetzt und bei 150 bis 170°C wasserdampfdestilliert. Es gehen insgesamt 1228 g Destillat über. Das im Destillat ausgefallene 2-Nitro-3,6-dichlorphenol wurde abfiltriert. Es wurden 148,6 g feuchtes Produkt mit einem Wassergehalt von 4,6 % und einer Reinheit von 95,4 % (nach GC) erhalten Dies entspricht einer Ausbeute von 86,6 % (100 %ig gerechnet). Der Schmelzpunkt betrug 46°C und die spektroskopischen Daten waren identisch mit denen aus Beispiel 1.

## Patentansprüche

1. Verfahren zur Herstellung von 2-Nitro-3,6-dichlorphenol durch Sulfonierung von 2,5-Dichlorphenol bei einer Temperatur von 70 bis 130°C und anschließende Nitrierung und Desulfonierung, dadurch gekennzeichnet, daß man die Nitrierung durch Zudosierung einer Nitriersäure aus 98 bis 100 %iger Salpetersäure und 96 bis 100 %iger Schwefelsäure, wobei die Schwefelsäure in der 1,5- bis 10fachen Gewichtsmenge bezogen auf die Salpetersäure eingesetzt wird, bei Temperaturen von -15 bis 35°C und einer Dosierzeit von mindestens 1 h durchführt.

2. Verfahren nach Anspruch 1, dadurch gekennzeichnet, daß die Salpetersäure und die Schwefelsäure 100 %ig eingesetzt werden.

3. Verfahren nach Anspruch 1 oder 2, dadurch gekennzeichnet, daß die Schwefelsäure in der 2- bis 8fachen, bevorzugt in der 4 bis 6fachen Gewichtsmenge, bezogen auf die Salpetersäure, eingesetzt wird.

4. Verfahren nach einem oder mehreren der Ansprüche 1 bis 3, dadurch gekennzeichnet, daß die Salpetersäure in einer Menge von 0,8 bis 1,3 mol, vorzugsweise von 0,9 bis 1,2 mol, besonders bevorzugt von 1,0 bis 1,1 mol pro mol 2,5-Dichlorphenol eingesetzt wird.

5. Verfahren nach einem oder mehreren derAnsprüche 1 bis 4, dadurch gekennzeichnet, daß die Nitrierung bei Temperaturen von 0 bis 25°C, vorzugsweise von 5 bis 20°C, durchgeführt wird.

6. Verfahren nach einem oder mehreren der Ansprüche 1 bis 5, dadurch gekennzeichnet, daß die Dosierzeit mindestens 1,5 h, vorzugsweise mindestens 2 h, beträgt.

7. Verfahren nach einem oder mehreren der Ansprüche 1 bis 6, dadurch gekennzeichnet, daß die Sulfonierung mit 98 bis 100 %iger, vorzugsweise mit 100 %iger Schwefelsäure, durchgeführt wird.

8. Verfahren nach Anspruch 7, dadurch gekennzeichnet, daß die Schwefelsäure in der 2,5 bis 12fachen, vorzugsweise in der 4 bis 8fachen Gewichtsmenge, bezogen auf 2,5-Dichlorphenol, eingesetzt wird.

9. Verfahren nach einem oder mehreren der Ansprüche 1 bis 8, dadurch gekennzeichnet, daß das bei der Nitrierung anfallende Reaktionsgemisch mit Wasser versetzt, bei Temperaturen von 150 bis 170°C wasserdampfdestilliert und das im Destillat anfallende 2-Nitro-3,6-dichlorphenol abfiltriert wird.

10. Verfahren nach einem oder mehreren der Ansprüche 1 bis 8, dadurch gekennzeichnet, daß das bei der Nitrierung anfallende Reaktionsgemisch mit Wasser versetzt, auf Temperaturen von 150 bis 170°C erhitzt und das ausgefallene 2-Nitro-3,6-dichlorphenol abfiltriert wird.

## Claims

1. A process for the preparation of 2-nitro-3,6-dichlorophenol by sulfonation of 2,5-dichlorophenol at a temperature of 70 to 130°C and subsequent nitration and desulfonation, which comprises carrying out the nitration by metering in a nitrating acid composed of 98 to 100% strength nitric acid and 96 to 100% strength sulfuric acid, the amount of sulfuric acid employed being 1.5 to 10 times the weight relative to the nitric acid, at temperatures from -15 to 35°C and a metering time of at least 1 hour.

2. The process as claimed in claim 1, wherein the strength of the nitric acid and the sulfuric acid is 100%.

3. The process as claimed in claim 1 or 2, wherein the amount of sulfuric acid employed is 2 to 8 times, preferably 4 to 6 times, the weight, relative to the nitric acid.

4. The process as claimed in one or more of claims 1 to 3, wherein the nitric acid is employed in an amount of 0.8 to 1.3 mol, preferably 0.9 to 1.2 mol, particularly preferably 1.0 to 1.1 mol, per mole of 2,5-dichlorophenol.

5. The process as claimed in one or more of claims 1 to 4, wherein the nitration is carried out at temperatures from 0 to 25°C, preferably 5 to 20°C.

6. The process as claimed in one or more of claims 1 to 5, wherein the metering time is at least 1.5 hours, preferably at least 2 hours.

7. The process as claimed in one or more of claims 1 to 6, wherein the sulfonation is carried out using 98 to 100% strength, preferably 100% strength, sulfuric acid.

8. The process as claimed in claim 7, wherein the amount of sulfuric acid employed is 2.5 to 12 times, preferably 4 to 8 times, the weight, relative to 2,5-dichlorophenol.

9. The process as claimed in one or more of claims 1 to 8, wherein the reaction mixture obtained in the nitration step is treated with water and subjected to steam distillation at temperatures from 150 to 170°C, and the 2-nitro-3,6-dichlorophenol, which is obtained in the distillate, is filtered off.

10. The process as claimed in one or more of claims 1 to 8, wherein the reaction mixture obtained in the nitration step is treated with water and heated at temperatures from 150 to 170°C, and the 2-nitro-3,6-dichlorophenol which has precipitated is filtered off.

## Revendications

1. Procédé pour la préparation de 2-nitro-3,6-dichlorophénol par sulfonation du 2,5-dichlorophénol à une température de 70 à 130°C, et nitration et désulfonation ultérieures, caractérisé en ce qu'on effectue la nitration par addition progressive d'un acide de nitration composé d'acide nitrique à 98-100 % et d'acide sulfurique à 96-100 %, l'acide sulfurique étant utilisé dans un excès de 1,5 à 10 fois la quantité en poids d'acide nitrique, à des températures de -15 à 35°C et un temps d'addition progressive d'au moins une heure.

2. Procédé selon la revendication 1, caractérisé en ce qu'on utilise l'acide nitrique et l'acide sulfurique à 100 %.

3. Procédé selon la revendication 1 ou 2, caractérisé en ce que l'acide sulfurique est utilisé dans un excès de 2 à 8 fois, de préférence de 4 à 6 fois en poids par rapport à l'acide nitrique.

4. Procédé selon une ou plusieurs des revendications 1 à 3, caractérisé en ce qu'on utilise l'acide nitrique en une quantité de 0,8 à 1,3 mole, de préférence de 0,9 à 1,2 mole, de manière particulièrement préférée de 1,0 à 1,1 mole par mole de 2,5-dichlorophénol.

5. Procédé selon une ou plusieurs des revendications 1 à 4, caractérisé en ce qu'on met en oeuvre la nitration à une température de 0 à 25°C, de préférence de 5 à 20°C.

6. Procédé selon une ou plusieurs des revendications 1 à 5, caractérisé en ce que le temps d'addition progressive est d'au moins 1,5 heure, de préférence 2 heures.

7. Procédé selon une ou plusieurs des revendications 1 à 6, caractérisé en ce qu'on met en oeuvre la sulfonation avec l'acide sulfurique à 98-100 %, de préférence à 100 %.

8. Procédé selon la revendication 7, caractérisé en ce qu'on utilise l'acide sulfurique dans un excès de 2,5 à 12 fois, de préférence de 4 à 8 fois en poids par rapport au 2,5-dichlorophénol.

9. Procédé selon une ou plusieurs des revendications 1 à 8, caractérisé en ce qu'on ajoute de l'eau au mélange réactionnel formé au cours de la nitration, on entraîne à la vapeur à des températures de 150 à 170°C et on sépare par filtration le 2-nitro-3,6-dichlorophénol formé dans le distillat.

10. Procédé selon une ou plusieurs des revendications 1 à 8, caractérisé en ce qu'on ajoute de l'eau au mélange réactionnel formé au cours de la nitration, on chauffe à une température de 150 à 170°C et on sépare par filtration le 2-nitro-3,6-dichlorophénol formé.
